# EUROPEAN PATENT APPLICATION

(11) **EP 4 850 661 A1**
(43) Date of publication of application: **09.12.2026**
(21) Application number: 25748353.7
(22) Date of filing: 17.01.2025
(51) Int. Cl.: A61K 8/39, A61K 8/86, A61K 8/90, A61Q 1/00, A61Q 19/08

(54) **SOFT FOCUS AGENT AND STRATUM CORNEUM SOFTENING AGENT**

(30) Priority: 31.01.2024 JP 2024012832
(71) Applicant: Shiseido Company, Ltd., Chuo-ku Tokyo 104-0061 (JP)
(72) Inventor: OKA, Takashi, Tokyo 104-0061 (JP); ITO, Yuji, Tokyo 104-0061 (JP)
(74) Representative: Ter Meer und Partner mbB
(86) International application number: PCT/JP2025/001290
(87) International publication number: WO 2025/164357

(57) **Abstract**

Provided are a novel soft focus agent and a novel stratum corneum softening agent.

A soft focus agent for the skin and a stratum corneum softening agent for the skin, wherein each of the soft focus agent and the stratum corneum softening agent comprises polymer (I) having a structure represented by formula (1) below as an active ingredient, or consists of polymer (I), wherein the polymer (I) has a structure represented by formula (1) below.

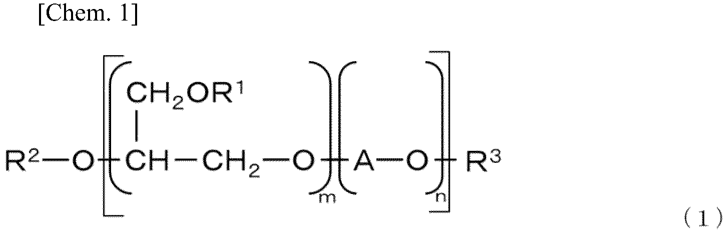

## Description

### FIELD

### [Field of the first invention]

The first invention relates to a soft focus agent for the skin.

### [Field of the second invention]

The second invention relates to a stratum corneum softening agent for the skin.

### BACKGROUND

### [Background of the first invention]

As a cosmetic having a soft focus effect, for example, PTL 1 reports a cosmetic composition containing (A) spherical silicone particles having protrusions chemically bonded to surfaces thereof, and (B) a polyorganosiloxane.

PTL 2 discloses, as soft focus agents, soft focus powders, which include polymethyl/methacrylate (PMMA); silica; hybrid pigments, for example, alumina-treated mica, titanium dioxide-treated talc, titanium dioxide-treated mica, and vinyl dimethicone/methicone silsesquioxane crosspolymer; alumina, barium sulfate; and synthetic mica, and silicone elastomers (for example, paragraph 0041).

### [Background of the second invention]

The stratum corneum tends to harden with age. Due to hardening of the stratum corneum, cracks may form in the skin. Hardening of the stratum corneum can also be a cause of wrinkles in the skin.

Therefore, it is important that the stratum corneum of the skin be kept soft.

To date, for example, PTL 3 reports a skin softening agent having a skin softening effect and primarily composed of a specific low-molecular-weight acetylated hyaluronic acid.

### [CITATION LIST]

### [PATENT LITERATURE]

[PTL 1] Japanese Unexamined Patent Publication (Kokai) No. 2008-137953
[PTL 2] Japanese Unexamined PCT Publication (Kohyo) No. 2008-521882
[PTL 3] Japanese Unexamined Patent Publication (Kokai) No. 09-071602

### SUMMARY

### [TECHNICAL PROBLEM]

### [Object of the first invention]

As disclosed in PTL 1 or 2 above, soft focus agents to date primarily use powders. A soft focus effect is often obtained by scattering reflected light from a powder in multiple directions.

The object of the first invention is to provide a novel soft focus agent.

### [Object of the second invention]

Development relating to stratum corneum softening agents for the skin remains necessary.

The object of the second invention is to provide a novel stratum corneum softening agent for the skin.

### [SOLUTION TO PROBLEM]

The first invention for achieving the above object is as follows.

### <Aspect 1-1>

A soft focus agent for skin, wherein
the soft focus agent comprises polymer (I) having a structure represented by formula (1) below as an active ingredient, or consists of the polymer (I), 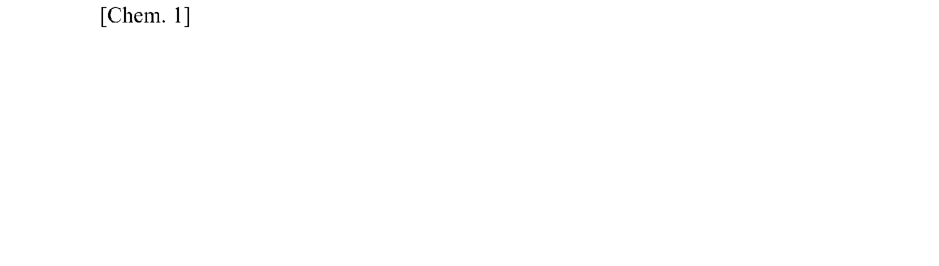 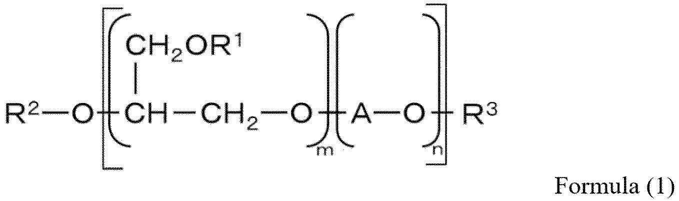
wherein, in the formula (1), R¹, R², and R³ are each independently a hydrogen atom or an alkyl group having 1 to 4 carbon atoms; A is an alkylene group having 2 to 4 carbon atoms; and m and n are each independently 1.0 to 50,
number average molecular weight of the polymer (I) is 10,000 or less, and
IOB value of the polymer (I) is 0.4 to 1.8.

### <Aspect 1-2>

The soft focus agent according to Aspect 1-1, wherein,
in the formula (1), A is represented by formula (2) below: 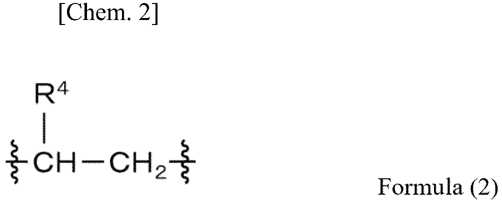
wherein, in the formula (2), R⁴ is an alkyl group having 1 or 2 carbon atoms.

### <Aspect 1-3>

The soft focus agent according to Aspect 1-1 or 1-2, wherein the polymer (I) is a random copolymer or a block copolymer.

### <Aspect 1-4>

The soft focus agent according to any one of Aspects 1-1 to 1-3, wherein, in the formula (1), m is 2 or greater, and at least a portion of R¹ is a methyl group.

### <Aspect 1-5>

The soft focus agent according to any one of Aspects 1-1 to 1-4, wherein, in the formula (1), at least one of R² and R³ is a methyl group.

### <Aspect 1-6>

A cosmetic composition, comprising the soft focus agent according to any one of Aspects 1-1 to 1-5.

The second invention for achieving the above object is as follows.

### <Aspect 2-1>

A stratum corneum softening agent for skin, wherein
the stratum corneum softening agent comprises polymer (I) having a structure represented by formula (1) below as an active ingredient, or consists of the polymer (I), 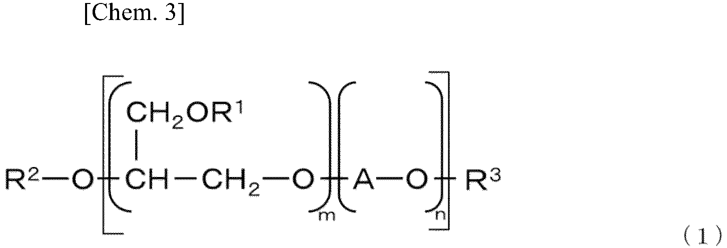
wherein, in the formula (1), R¹, R², and R³ are each independently a hydrogen atom or an alkyl group having 1 to 4 carbon atoms; A is an alkylene group having 2 to 4 carbon atoms; and m and n are each independently 1.0 to 50,
number average molecular weight of the polymer (I) is 10,000 or less, and
IOB value of the polymer (I) is 0.4 to 1.8.

### <Aspect 2-2>

The stratum corneum softening agent according to Aspect 2-1, wherein,
in the formula (1), A is represented by formula (2) below: 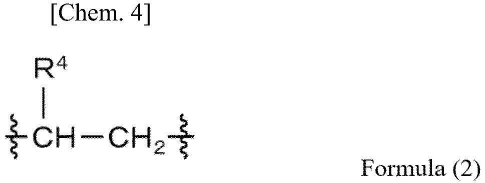
wherein, in the formula (2), R⁴ is an alkyl group having 1 or 2 carbon atoms.

### <Aspect 2-3>

The stratum corneum softening agent according to Aspect 2-1 or 2-2, wherein the polymer (I) is a random copolymer or a block copolymer.

### <Aspect 2-4>

The stratum corneum softening agent according to any one of Aspects 2-1 to 2-3, wherein, in the formula (1), m is 2 or greater, and at least a portion of R¹ is a methyl group.

### <Aspect 2-5>

The stratum corneum softening agent according to any one of Aspects 2-1 to 2-4, wherein, in the formula (1), at least one of R² and R³ is a methyl group.

### <Aspect 2-6>

A cosmetic composition, comprising the stratum corneum softening agent according to any one of Aspects 2-1 to 2-5.

### [ADVANTAGEOUS EFFECTS OF INVENTION]

### [Effect of the first invention]

According to the first invention, a novel soft focus agent can be provided.

### [Effect of the second invention]

According to the second invention, a novel stratum corneum softening agent for the skin can be provided.

### BRIEF DESCRIPTION OF DRAWINGS

FIG. 1 is a chart showing results of haze of the compositions of Examples 1-3 and 1-4 and Comparative Example 1-2.
FIG. 2 is a view showing soft focus effects of the compositions of Examples 1-3 and 1-4 and Comparative Example 1-2, using a Beaulax pore model.
FIG. 3 is a chart showing storage modulus ratios of each of Examples 2-1 and 2-2 and Comparative Example 2-1 relative to Comparative Example 2-1.

### DESCRIPTION OF EMBODIMENTS

### [The first invention]

Hereinafter, embodiments of the first invention will be described in detail. The first invention is not limited to the following embodiments, and various modifications can be made within the spirit of the present invention.

### <<Soft focus agent>>

The soft focus agent of the first invention is
a soft focus agent for the skin, wherein
the soft focus agent comprises polymer (I) having a structure represented by formula (1) below as an active ingredient, or consists of the polymer (I), 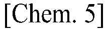 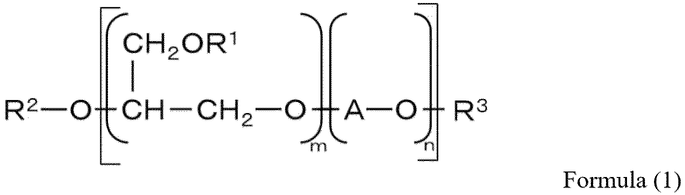
wherein, in formula (1), R¹, R², and R³ are each independently a hydrogen atom or an alkyl group having 1 to 4 carbon atoms; A is an alkylene group having 2 to 4 carbon atoms; and m and n are each independently 1.0 to 50,
the number average molecular weight of the polymer (I) is 10,000 or less, and
the IOB value of the polymer (I) is 0.4 to 1.8.

The soft focus agent of the first invention is an unprecedented and novel agent, and can be applied to the skin to impart a soft focus effect. Without being bound by theory, the reason therefor is presumably that at least a portion of polymer (I) of the first invention, due to the unique structure and characteristics thereof, can exert a scattering effect on the surface layer of the skin.

In the first invention, soft focus agent refers to a cosmetic base having the effect of making skin discoloration such as blemishes, freckles, and dullness and skin unevenness such as pores and wrinkles less visible (soft focus effect).

In the first invention, the soft focus effect can be evaluated by measuring haze using a haze measurement device. A higher haze value can be determined as having a greater soft focus effect. Haze can be determined by the following mathematical formula (a).
[Math. 1] $Haze \left(%\right) = \left(Td/Tt\right) \times 100$ (a)

In the formula: Td: diffused light transmittance (%), Tt: total light transmittance (%).

The soft focus agent of the first invention comprises polymer (I) as an active ingredient, or consists of the polymer (I). Hereinafter, polymer (I) of the first invention will be described in detail.

### <Polymer (I) of the first invention>

Polymer (I) of the first invention has a structure represented by formula (1) below.

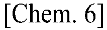

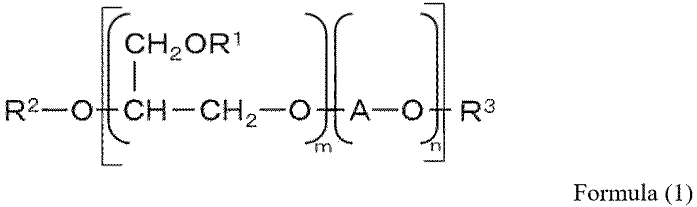

In formula (1), R¹, R², and R³ are each independently a hydrogen atom or an alkyl group having 1 to 4 carbon atoms. In the first invention, examples of the alkyl group having 1 to 4 carbon atoms include methyl group, ethyl group, n-propyl group, isopropyl group, n-butyl group, sec-butyl group, and tert-butyl group. As long as the effect of the first invention is not impaired, the alkyl group may further have a substituent. The substituent is not particularly limited, and examples thereof include halogen groups.

According to one embodiment of the first invention, in formula (1), R¹, R², and R³ may each independently be a hydrogen atom, a methyl group, an ethyl group, an n-propyl group, an isopropyl group, an n-butyl group, a sec-butyl group, or a tert-butyl group.

According to one embodiment of the first invention, in formula (1), it is preferable that R¹ be a hydrogen atom, a methyl group, or an ethyl group. When m is 2 or greater, i.e., when a plurality of R¹ are present, each R¹ may be the same or different. Particularly, it is preferable that at least a portion of R¹ be a methyl group. The case where at least a portion of R¹ is a methyl group is preferable from the viewpoint of improving feel during use.

According to one embodiment of the first invention, in formula (1), R¹ may be in a state where hydrogen atoms and alkyl groups having 1 to 4 carbon atoms coexist. Specifically, one portion of R¹ may be hydrogen atoms, and the other portion may be alkyl groups having 1 to 4 carbon atoms. Preferably, one portion of R¹ is hydrogen atoms, and the other portion is methyl groups.

According to one embodiment of the first invention, in formula (1), it is preferable that R² be a hydrogen atom, a methyl group, or an ethyl group. Particularly, a methyl group is more preferable.

According to one embodiment of the first invention, in formula (1), R² may be in a state where a hydrogen atom and an alkyl group having 1 to 4 carbon atoms coexist. Specifically, one portion of R² may be hydrogen atoms, and the other portion may be alkyl groups having 1 to 4 carbon atoms. Preferably, one portion of R² is hydrogen atoms, and the other portion is methyl groups. When a plurality of R¹ and R² are present and at least a portion thereof is a methyl group, the ratio of methyl group to hydrogen (CH₃/H) in R¹ and R² is not particularly limited, and for example, may be 0.05 or greater, 0.10 or greater, 0.15 or greater, 0.20 or greater, 0.25 or greater, 0.30 or greater, or 0.35 or greater, and may be 0.99 or less, 0.90 or less, 0.80 or less, 0.70 or less, or 0.60 or less.

According to one embodiment of the first invention, in formula (1), it is preferable that R³ be a hydrogen atom, a methyl group, or an ethyl group. Particularly, it is more preferable that R³ be a methyl group. When a plurality of R³ are present and at least a portion thereof is a methyl group, conforming with R¹ and R² described above, the ratio of methyl group to hydrogen (CH₃/H) in R¹, R², and R³ is not particularly limited, and for example, may be 0.05 or greater, 0.10 or greater, 0.15 or greater, 0.20 or greater, 0.25 or greater, 0.30 or greater, or 0.35 or greater, and may be 0.99 or less, 0.90 or less, 0.80 or less, 0.70 or less, or 0.60 or less.

According to one embodiment of the first invention, in formula (1), it is preferable that at least one of R² and R³ be a methyl group.

In formula (1), A is an alkylene group having 2 to 4 carbon atoms. More specifically, A may be, for example, an ethylene group, a propylene group, a butylene group, or an isobutylene group, but is not limited thereto. Particularly, it is preferable that A be represented by formula (2) below.

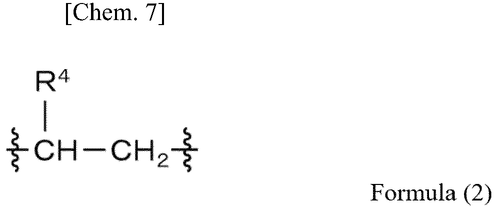

In formula (2), R⁴ is an alkyl group having 1 or 2 carbon atoms. More specifically, R⁴ may be a methyl group or an ethyl group.

In formula (1), m and n represent the average number of moles added for each structural unit. The m and n are each independently 1.0 to 50, and more specifically, may be 1.0 or greater, 1.5 or greater, 2.0 or greater, 3.0 or greater, 4.0 or greater, 5.0 or greater, 6.0 or greater, 7.0 or greater, 8.0 or greater, 9.0 or greater, 10 or greater, 15 or greater, or 20 or greater, and may be 50 or less, 45 or less, 40 or less, 35 or less, 34 or less, 32 or less, 30 or less, 28 or less, 26 or less, 25 or less, 24 or less, 22 or less, 20 or less, 18 or less, 16 or less, 15 or less, 14 or less, 12 or less, 10 or less, or 5.0 or less.

In formula (1), m is preferably 1.0 or greater and 25 or less, 1.0 or greater and 20 or less, or 1.0 or greater and 14 or less, and more preferably 2.0 or greater and 20 or less, 2.0 or greater and 15 or less, or 2.0 or greater and 5.0 or less. In formula (1), n is preferably 2.0 or greater and 40 or less, 2.0 or greater or 35 or less, or 2.0 or greater and 34 or less, and more preferably 6.0 or greater and 35 or less, or 6.0 or greater and 12 or less.

According to one embodiment of the first invention, in formula (1), m + n may be 4.0 or greater, 4.5 or greater, 5.0 or greater, 6.0 or greater, 7.0 or greater, 8.0 or greater, 9.0 or greater, 10 or greater, 11 or greater, 12 or greater, 13 or greater, 14 or greater, 15 or greater, or 17 or greater, or may be 55 or less, 53 or less, 50 or less, 45 or less, 42 or less, 40 or less, 35 or less, 30 or less, 25 or less, 20 or less, 15 or less, or 12 or less.

According to one embodiment of the first invention, in formula (1), m:n may be 1:10 to 10:1, 1:6 to 6:1, or 1:5 to 5:1.

For polymer (I) of the first invention, the number average molecular weight is 10,000 or less. More specifically, the number average molecular weight of polymer (I) of the first invention, for example, may be 10,000 or less, 5,000 or less, 3,500 or less, 3,000 or less, 2,800 or less, 2,600 or less, 2,500 or less, 2,000 or less, 1,500 or less, 1,400 or less, 1,300 or less, 1,200 or less, 1,100 or less, 1,000 or less, 900 or less, 800 or less, 700 or less, or 600 or less, and may be 130 or greater, 150 or greater, 200 or greater, 250 or greater, 300 or greater, 350 or greater, 400 or greater, 450 or greater, or 500 or greater. The number average molecular weight of polymer (I) of the first invention is preferably 300 or greater and 3,500 or less, and more preferably 500 or greater and 3,500 or less, or 500 or greater and 1,200 or less.

The IOB value of polymer (I) of the first invention is 0.4 to 1.8. More specifically, the IOB value of polymer (I) of the first invention, for example, may be 0.4 or greater, 0.5 or greater, 0.6 or greater, 0.7 or greater, 0.8 or greater, or 0.9 or greater, and may be 1.8 or less, 1.6 or less, 1.4 or less, 1.2 or less, or 1.0 or less. The IOB value of polymer (I) of the first invention is preferably 0.7 or greater and 1.2 or less.

IOB value is an abbreviation for inorganic/organic balance, is a value that represents a ratio of inorganic value to organic value, and acts as an indicator showing the degree of polarity of an organic compound. The IOB value, specifically, is represented as IOB value = inorganic value / organic value. With respect to each of "inorganic value" and "organic value," the "inorganic value" and "organic value" are set depending on various atoms or functional groups, such that, for example, the "organic value" for one carbon atom in a molecule is 20, and the "inorganic value" for one hydroxyl group is 100, and by summing the "inorganic values" and "organic values" of all atoms and functional groups in an organic compound, the IOB value of the organic compound can be calculated (refer to, for example, Yoshio Koda, Organic Conceptual Diagrams -Fundamentals and Applications-, pp. 11 to 17, Sankyo Publishing Co., Ltd., published in 1984). Regarding the method for determining IOB values, the method described in the Examples can be referenced.

Polymer (I) of the first invention may be a block copolymer, or may be a random copolymer.

The production method for polymer (I) of the first invention is not particularly limited, and for example, may be carried out by addition polymerization of epoxides corresponding to each structural unit. The addition polymerization may be block polymerization, or may be random polymerization.

The production method for polymer (I) of the first invention may further comprise further reacting an alkyl halide with the polymer obtained by the above addition polymerization. A hydroxyl group in a molecule of the polymer obtained via the reaction with an alkyl halide is blocked by the alkyl group, and as a result, hydrophobicity of the entirety of the polymer can be adjusted as desired. In the present invention, blocking rate of hydroxyl group by alkyl group within a molecule of the polymer, for example, may be 20% or greater, 30% or greater, 35% or greater, 40% or greater, 45% or greater, 50% or greater, or 55% or greater, and may be 60% or less, 59% or less, 58% or less, 57% or less, 56% or less, or 55% or less. The blocking rate of hydroxyl group by alkyl group can be determined according to the method described in the Examples.

Polymer (I) of the first invention, as one of the characteristics thereof, has both high water solubility and high lipid solubility.

The degree of solubility in water of polymer (I) of the first invention can be, for example, 5% by mass or more, 10% by mass or more, 20% by mass or more, 30% by mass or more, 40% by mass or more, or 50% by mass or more.

As an indicator of lipid solubility of polymer (I) of the first invention, for example, degree of solubility in olive oil can be used. The degree of solubility in olive oil of polymer (I) of the first invention, for example, can be 0.01% by mass or more, 0.05% by mass or more, 0.10% by mass or more, 0.50% by mass or more, or 1.00% by mass or more. The upper limit value of degree of solubility in olive oil of polymer (I) of the first invention is not particularly limited, and for example, may be 10% by mass or less.

The soft focus agent of the first invention may comprise polymer (I) of the first invention as an active ingredient, or may consist of polymer (I) of the first invention. Therefore, the content of polymer (I) of the first invention, when the soft focus agent of the first invention comprises polymer (I) of the first invention as an active ingredient, is not particularly limited, and for example, may be 50% by mass or more, 60% by mass or more, 70% by mass or more, 80% by mass or more, 90% by mass or more, 95% by mass or more, 99% by mass or more, or 100% by mass, and may be 99% by mass or less, relative to the entirety of the soft focus agent.

### <<Cosmetic composition>>

The first invention also provides a cosmetic composition comprising the soft focus agent of the first invention.

The content of the soft focus agent of the first invention in the cosmetic composition of the first invention is not particularly limited, and for example, the soft focus agent of the first invention in the entirety of a cosmetic may be in an amount of 0.001 to 90% by mass, 0.1 to 50% by mass, 0.1 to 20% by mass, or 0.1 to 10% by mass. More specifically, for example, the soft focus agent of the first invention may be 0.001% by mass or more, 0.005% by mass or more, 0.01% by mass or more, 0.05% by mass or more, 0.1% by mass or more, 0.5% by mass or more, 1.0% by mass or more, 1.5% by mass or more, or 2.0% by mass or more, and may be 95% by mass or less, 90% by mass or less, 80% by mass or less, 70% by mass or less, 60% by mass or less, 50% by mass or less, 40% by mass or less, 30% by mass or less, 20% by mass or less, 10% by mass or less, 9.0% by mass or less, 8.0% by mass or less, 7.0% by mass or less, 6.0% by mass or less, 5.0% by mass or less, 4.0% by mass or less, 3.0% by mass or less, 2.0% by mass or less, or 1.0% by mass or less, relative to the entirety of the cosmetic.

The cosmetic composition of the first invention may be used in a form such as liquid, emulsion, cream, powder, foam, or solid. The cosmetic composition of the first invention, for example, may be a solution system, a solubilized system, an emulsion system, a powder dispersion system, a water-oil two-layer system, a water-oil-powder three-layer system, a gel, a mist, a spray, a mousse, a roll-on, or a stick, or may be a preparation obtained by impregnating a sheet such as a nonwoven fabric with the composition or applying the composition thereto.

The cosmetic composition of the first invention is preferably used as a cosmetic for which a soft focus effect is particularly required, and/or as a cosmetic for which correction of skin unevenness such as pores and fine lines and improvement of skin texture after cosmetic application is required.

The cosmetic for which a soft focus effect is required and/or improvement of skin texture after cosmetic application is required is not particularly limited, and examples thereof include makeup cosmetics such as foundations, concealers, blushes, face powders (loose powders and pressed powders), color correctors, cosmetic primers, BB creams, eye colors, and lipsticks; and skin care cosmetics such as emulsions, creams, serums, day creams, and sunscreens.

These cosmetics may generally contain, depending on the intended use or mode of application, cosmetic ingredients such as film-forming polymers, surfactants, thickeners, water, moisturizers, fragrances, pigments or dyes, water-repellent oil components such as silicone oils, ultraviolet absorbers, astringents, cooling agents, anti-inflammatory agents, whiteners, various extracts, and plant and seaweed extracts in appropriate amounts, but are not limited to these ingredients.

### [The second invention]

Hereinafter, embodiments of the second invention will be described in detail. The second invention is not limited to the following embodiments, and various modifications can be made within the spirit of the present invention.

### <<Stratum corneum softening agent>>

The stratum corneum softening agent of the second invention is
a stratum corneum softening agent for the skin, wherein
the stratum corneum softening agent comprises polymer (I) having a structure represented by formula (1) below as an active ingredient, or consists of the polymer (I), 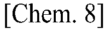 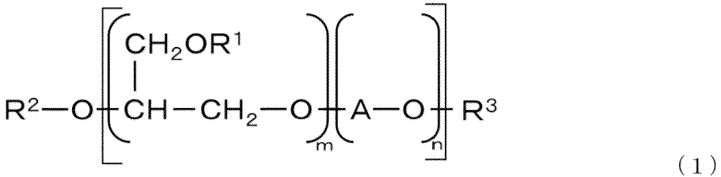
wherein, in formula (1), R¹, R², and R³ are each independently a hydrogen atom or an alkyl group having 1 to 4 carbon atoms; A is an alkylene group having 2 to 4 carbon atoms; and m and n are each independently 1.0 to 50,
the number average molecular weight of the polymer (I) is 10,000 or less, and
the IOB value of the polymer (I) is 0.4 to 1.8.

The stratum corneum softening agent of the second invention is an unprecedented and novel agent, and can be applied to the skin to impart a softening effect. Without being bound by theory, the reason therefor is considered to be the amphiphilicity of polymer (I) of the second invention from the unique structure thereof. The polymer (I) being amphiphilic is considered to easily penetrate the stratum corneum of the skin, thereby enabling interaction with keratin in the stratum corneum (for example, disrupting hydrogen bonds between keratin molecules), and as a result, softness of the stratum corneum is presumably enhanced.

The stratum corneum softening agent of the second invention is expected to have an effect associated with improving skin wrinkles by softening the stratum corneum.

According to the mechanism of the hypothesis described above, it can be said that the skin softening effect of the stratum corneum softening agent of the second invention differs from the skin softening effect of certain moisturizers.

For example, PTL 1 described above discloses a skin softening effect of a specific low-molecular-weight acetylated hyaluronic acid. The mechanism of the skin softening effect in this case is considered to be as follows. That is, the specific low-molecular-weight acetylated hyaluronic acid has excellent affinity for the skin, and can be retained on the skin surface. Therefore, presumably, moisture evaporating from the stratum corneum interior can be captured on the skin surface, and as a result, a softening effect of the skin can be maintained. However, not all moisturizers having a moisturizing effect can necessarily exhibit the stratum corneum softening effect as described in PTL 1 above.

### <Polymer (I) of the second invention>

The stratum corneum softening agent of the second invention comprises polymer (I) as an active ingredient, or consists of the polymer (I). Since polymer (I) of the second invention is the same as "polymer (I) of the first invention" described above, the detailed description thereof is omitted. In the description of "polymer (I) of the first invention," the part relating to "the first invention" is replaced with "the second invention" as appropriate.

In the second invention, the stratum corneum softening agent of the second invention may comprise polymer (I) of the second invention as an active ingredient, or may consist of polymer (I) of the second invention. Therefore, the content of polymer (I) of the second invention, when the stratum corneum softening agent of the second invention comprises polymer (I) of the second invention as an active ingredient, is not particularly limited, and for example, may be 50% by mass or more, 60% by mass or more, 70% by mass or more, 80% by mass or more, 90% by mass or more, 95% by mass or more, 99% by mass or more, or 100% by mass, and may be 99% by mass or less, relative to the entirety of the stratum corneum softening agent.

### <<Cosmetic composition>>

The second invention also provides a cosmetic composition comprising the stratum corneum softening agent of the second invention.

The content of the stratum corneum softening agent of the second invention in the cosmetic composition of the second invention is not particularly limited, and for example, the stratum corneum softening agent of the second invention in the entirety of a cosmetic may be in an amount of 0.001 to 90% by mass, 0.1 to 50% by mass, 0.1 to 20% by mass, or 0.1 to 10% by mass. More specifically, for example, the stratum corneum softening agent of the second invention may be 0.001% by mass or more, 0.005% by mass or more, 0.01% by mass or more, 0.05% by mass or more, 0.1% by mass or more, 0.5% by mass or more, 1.0% by mass or more, 1.5% by mass or more, or 2.0% by mass or more, and may be 95% by mass or less, 90% by mass or less, 80% by mass or less, 70% by mass or less, 60% by mass or less, 50% by mass or less, 40% by mass or less, 30% by mass or less, 20% by mass or less, 10% by mass or less, 9.0% by mass or less, 8.0% by mass or less, 7.0% by mass or less, 6.0% by mass or less, 5.0% by mass or less, 4.0% by mass or less, 3.0% by mass or less, 2.0% by mass or less, or 1.0% by mass or less, relative to the entirety of the cosmetic.

The cosmetic composition of the second invention may optionally further comprise various ingredients, in addition to the stratum corneum softening agent of the second invention.

Examples of the ingredients include, but are not limited to, oils such as liquid paraffin, squalane, lanolin derivatives, higher alcohols, various ester oils, silicone oils, polyalkylene glycol polyethers, other carboxylic acids, oligoester compounds, and terpene-based hydrocarbon oils; surfactants, ultraviolet absorbers, ultraviolet scattering agents; resins such as acrylic resins, silicone resins, and polyvinyl pyrrolidone; proteins and protein hydrolysates such as soybean protein, gelatin, collagen, silk fibroin, and elastin; preservatives such as ethylparaben and butylparaben; activators such as biotin and pantothenic acid derivatives; diluents such as ethanol, isopropanol, and tetrachlorodifluoroethane, toluene; thickeners such as carboxyvinyl polymers; chelating agents, antioxidants, moisturizers; active agents such as tranexamic acid, nicotinamide, potassium 4-methoxysalicylate, retinol, and retinol acetate; fragrances, and coloring agents.

The cosmetic composition of the second invention may be used in a form such as liquid, emulsion, cream, powder, foam, or solid. The cosmetic composition of the second invention, for example, may be a solution system, a solubilized system, an emulsion system, a powder dispersion system, a water-oil two-layer system, a water-oil-powder three-layer system, a gel, a mist, a spray, a mousse, a roll-on, or a stick, or may be a preparation obtained by impregnating a sheet such as a nonwoven fabric with the composition or applying the composition thereto.

### EXAMPLES

With reference to the Examples below, the first and second inventions will be further described in detail. However, the first and second inventions are not limited thereto.

### [The first invention]

### <<Synthesis Examples 1 and 2>>

In each of the following Synthesis Examples 1 and 2, a polymer having a structure represented by formula (3) below was synthesized.

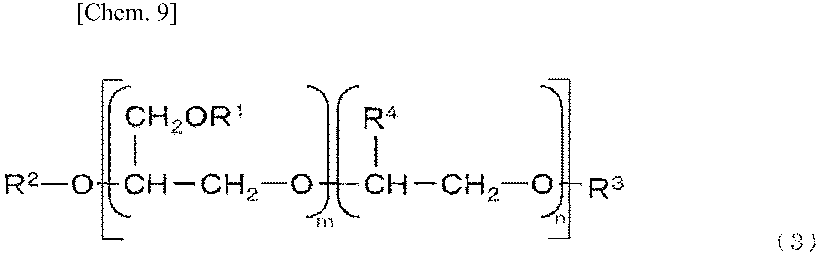

In formula (3), the portion within [ ] is a random copolymer.

### <Synthesis Example 1>

Synthesis Example 1: polymer 1, where R¹ = H, R² = H, R³ = CH₃, R⁴ = CH₃, m = 2.0, and n = 6.0

Synthesis was carried out according to the method below to obtain polymer 1.

128 g of methanol and, as a catalyst, 6.4 g of potassium hydroxide were placed inside an autoclave, air inside the autoclave was replaced with dry nitrogen, and the catalyst was completely dissolved at R0°C while stirring. Next, a mixture of 592 g of glycidol and 1392 g of propylene oxide was added dropwise over 20 h at 95°C with a dropping apparatus, followed by stirring for 5 h. The reaction composition was taken out from the autoclave, neutralized with hydrochloric acid to a pH of 6 to 7, and treated under reduced pressure of -0.095 MPa (50 mmHg) at 100°C for 1 h to remove contained moisture. After the treatment, filtration was further carried out to remove any salt generated, and 2000 g of polymer 1 of Synthesis Example 1 was obtained.

Hereinafter, values of various physical properties were determined with respect to the obtained polymer 1.

### (Number average molecular weight)

The number average molecular weight of the obtained polymer 1 was calculated by gel permeation chromatography (GPC) measurement. Using a SHODEX(registered trademark) GPC101 dedicated GPC system as the system, a SHODEX RI-71s as the differential refractometer, a SHODEX KF-G as the guard column, and three SHODEX KF804L installed in series as columns, tetrahydrofuran as the developing solvent was flowed at a flow rate of 1 ml/min at a column temperature of 40°C. 0.1 ml of a 0.1 wt% tetrahydrofuran solution of the resulting reaction product was injected, and a chromatogram represented by refractive index intensity and elution time was obtained using a BORWIN GPC calculation program. A number average molecular weight was determined from the chromatogram using polyethylene glycol as a reference, and was approximately 530.

### (Weight average molecular weight)

The weight average molecular weight of the obtained polymer 1, determined by GPC calculation in the same manner as described above, was approximately 859.

### (Polydispersity)

From the results of number average molecular weight and weight average molecular weight determined above, the polydispersity Mw/Mn of polymer 1 was 1.62.

### (IOB value)

The IOB value of the obtained polymer 1 was determined as follows, and was 1.1.

More specifically, in polymer 1, the organic value was calculated based on 20 for carbon and -10 for an iso-branch. The inorganic value was calculated based on 100 for a hydroxyl group and 20 for an ether bond.
- Methanol site: (1 carbon atom, 1 hydroxyl group) × 1
   Organic value: 20
   Inorganic value: 100
- Glycidol site: (3 carbon atoms, 1 iso-branch, 1 hydroxyl group, 1 ether bond) × 2
   Organic value: (60 - 10) × 2 = 100
   Inorganic value: (100 + 20) × 2 = 240
- Propylene oxide site: (3 carbon atoms, 1 iso-branch, 1 ether bond) × 6
   Organic value: (60 - 10) × 6 = 300
   Inorganic value: 20 × 6 = 120

From the above, the total organic value is 420 and the total inorganic value is 460, and thus the IOB value is 1.09.

### (Cloud point)

A 5 wt% aqueous solution of the obtained polymer 1 was prepared, heated to 85°C, and then cooled. The temperature at which a transparent solution was formed was 74°C. Thus, it was found that the cloud point of polymer 1 was 74°C.

### (Hydroxyl value)

The hydroxyl value of the obtained polymer 1 was measured in accordance with the measurement method in JIS K-1557-1, and was 294.

### (Solubility in water)

The solubility in water of the obtained polymer 1 was determined by the same method as that of "Compatibility evaluation" described below, and was 50% by mass or more.

### (Solubility in olive oil)

The solubility in olive oil of the obtained polymer 1 was determined as follows. Specifically, polymer 1 was mixed with olive oil so as to obtain a concentration of 1.0 wt%, and appearance was confirmed. "Yes" was indicated if appearance was uniform. In this case, the solubility in olive oil of polymer 1 was 1.0% by mass.

The values of various physical properties of polymer 1 measured above are shown in Table 1-1 below.

### <Synthesis Example 2>

Synthesis Example 2: polymer 2, where R¹ = H and CH₃, R² = H and CH₃, R³ = CH₃, R⁴ = CH₃, m = 2.0, and n = 6.0

Synthesis was carried out according to the method below to obtain polymer 2.

128 g of methanol and, as a catalyst, 6.4 g of potassium hydroxide were placed inside an autoclave, air inside the autoclave was replaced with dry nitrogen, and the catalyst was completely dissolved at 80°C while stirring. Next, a mixture of 592 g of glycidol and 1392 g of propylene oxide was added dropwise over 20 h at 95°C with a dropping apparatus, followed by stirring for 2 h. Next, 244 g of potassium hydroxide was added, and the inside of the system was replaced with dry nitrogen. After the inside of the system was replaced with dry nitrogen, 200 g of methyl chloride was injected at a temperature of 80 to 130°C and reacted for 5 h. The reaction composition was then taken out from the autoclave, neutralized with hydrochloric acid to a pH of 6 to 7, and treated under reduced pressure of -0.095 MPa (50 mmHg) at 100°C for 1 h to remove contained moisture. After the treatment, filtration was further carried out to remove any salt generated, and 1820 g of polymer 2 was obtained. A sample was taken before reacting the methyl chloride, and the hydroxyl value of the purified polymer was 125. Thus, it was found that the ratio of methyl group to hydrogen atom (CH₃/H) in R² and R³ was 0.57. Specifically, the hydroxyl group blocking rate of polymer 2 was 57%.

In the same manner as in polymer 1 described above, values of various physical properties of the obtained polymer 2 were determined, and the results are shown in Table 1-1 below.

**[Table 1-1]**

| (Table 1-1 Synthesis Example 1 and Synthesis Example 2) | | |
|---|---|---|
| Synthesis Example No. (Polymer No.) | 1 | 2 |
| | 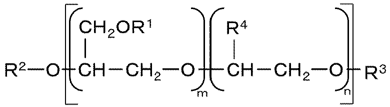 | |
| R¹ | H | CH₃ and H |
| R² | H | CH₃ and H |
| R³ | CH3 | CH3 |
| R⁴ | CH3 | CH3 |
| Ratio of CH₃ to H (CH₃/H) in R¹ and R² | - | 0.57 |
| m | 2.0 | 2.0 |
| n | 6.0 | 6.0 |
| m + n | 8.0 | 8.0 |
| Polymerization type | Random | Random |
| Number average molecular weight | 530 | 673 |
| Weight average molecular weight | 859 | 956 |
| Polydispersity (Mw/Mn) | 1.62 | 1.42 |
| IOB value | 1.09 | 0.77 |
| Hydroxyl blocking rate (%) | - | 57 |
| Hydroxyl value (mgKOH/g) | 294 | 125 |
| Cloud point (5% aq.) | 74°C | 45°C |
| Solubility (water) | 50% by mass or more | 30% by mass |
| Solubility (oil) _{*}1 | Yes | Yes |

| | | |
|---|---|---|
| _{*}1 Synthesized compound was mixed with olive oil so as to obtain a concentration of 1.0% by mass, and appearance was confirmed. "Yes" was indicated if appearance was uniform. | | |

### <Compatibility evaluation>

Polymers 1 and 2 synthesized above were each mixed with water and various oils at certain concentrations, stirred, and then left to stand for one day, followed by examining individual compatibility by visual observation. The results are shown in Table 1-2 below. In Table 1-2, "Yes" represents "compatible" and "No" represents "incompatible."

### [Table 1-2]

**(Table 1-2)**

| | Concentration of each polymer (% by mass) | Polymer 1 | Polymer 2 |
|---|---|---|---|
| Water | 30 | Yes | Yes |
| | 50 | Yes | No |
| Squalane | 1 | No | No |
| Hydrogenated polyisobutene | 1 | No | No |
| Olive oil | 1 | Yes | Yes |
| Cetyl ethylhexanoate | 1 | No | No |
| Macadamia seed oil | 1 | Yes | Yes |
| Isopropyl palmitate | 1 | No | Yes |
| Isopropyl myristate | 1 | No | Yes |
| Ethylhexyl palmitate | 1 | No | No |

### <<Examples 1-1 and 1-2 and Comparative Example 1-1>>

In Examples 1-1 and 1-2, 5% by mass aqueous solutions of polymers 1 and 2 obtained above, respectively, were prepared, and the soft focus effect was evaluated. In Comparative Example 1-1, 100% water was used.

### (Evaluation of soft focus effect)

Haze value (cloudiness value) was measured using a haze meter manufactured by Murakami Color Research Laboratory.

More specifically, before applying each sample, the haze of the human stratum corneum was measured. The 5% by mass aqueous solution of each sample was then applied, and the haze value was remeasured after 24 h to compare the haze before and after application. The results are shown in Table 1-3.

### [Table 1-3]

**(Table 1-3)**

| | Polymer (I) | Haze [unit: %] | |
|---|---|---|---|
| | | Before application | 24 h after application |
| Comparative Example 1-1 | - | 63.0 | 63.1 |
| Example 1-1 | Polymer 1 | 65.9 | 67.6 |
| Example 1-2 | Polymer 2 | 65.1 | 66.7 |

As is apparent from the results in Table 1-3, it was found that the aqueous solutions of Examples 1-1 and 1-2 both showed a higher haze compared to Comparative Example 1-1.

Specifically, it was found that the aqueous solutions of Examples 1-1 and 1-2 comprising polymers 1 and 2, respectively, exhibited a soft focus effect. In other words, it was suggested that polymers 1 and 2 could each be used as a soft focus agent.

### <<Examples 1-3 and 1-4 and Comparative Example 1-2>>

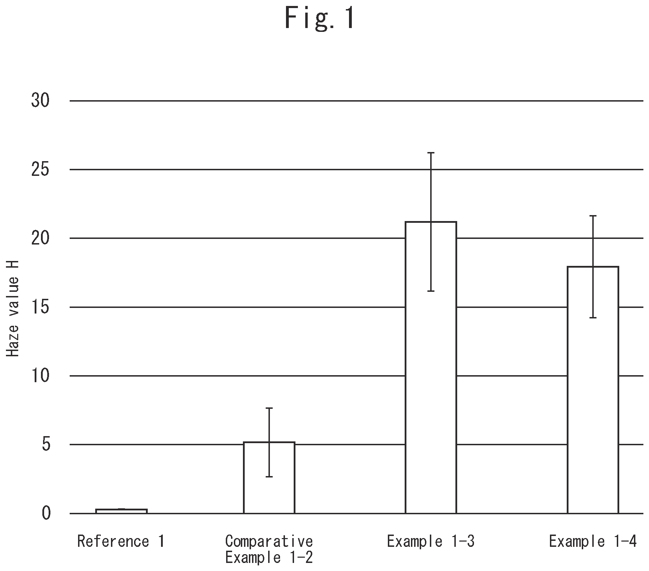

Based on the formulations in Table 1-4, cosmetic compositions of Examples 1-3 and 1-4 and Comparative Example 1-2 were prepared. Each of the obtained compositions was applied with a doctor blade (glass plate, thickness of 0.075 mm), and haze was measured after 24 h using a haze meter manufactured by Murakami Color Research Laboratory. As Reference 1, the haze of the used doctor blade was measured. The obtained results are shown in FIG. 1.

### [Table 1-4]

**(Table 1-4)**

| Component | | Example 1-3 | Example 1-4 | Comparative Example 1-2 |
|---|---|---|---|---|
| Polymer (I) of the first invention | Polymer 1 | 5.0 | - | - |
| | Polymer 2 | - | 5.0 | - |
| Ion-exchanged water | | 86.09 | 86.09 | 91.09 |
| EDTA 2Na-2H₂O | | 0.05 | 0.05 | 0.05 |
| Carbomer | | 0.16 | 0.16 | 0.16 |
| Acrylates/alkyl acrylate crosspolymer | | 0.1 | 0.1 | 0.1 |
| Olefin oligomer | | 5.0 | 5.0 | 5.0 |
| Ethanol | | 3.0 | 3.0 | 3.0 |
| Phenoxyethanol | | 0.5 | 0.5 | 0.5 |
| Caustic potash | | 0.1 | 0.1 | 0.1 |
| Total | | 100 | 100 | 100 |

| | | | | |
|---|---|---|---|---|
| *The unit of content for each component is "% by mass." | | | | |

As is apparent from the results in FIG. 1, it was found that the compositions of Examples 1-3 and 1-4 comprising polymers 1 and 2, respectively, exhibited a markedly greater soft focus effect compared to Comparative Example 1-2 and Reference 1.

### <Evaluation of soft focus effect using Beaulax pore model>

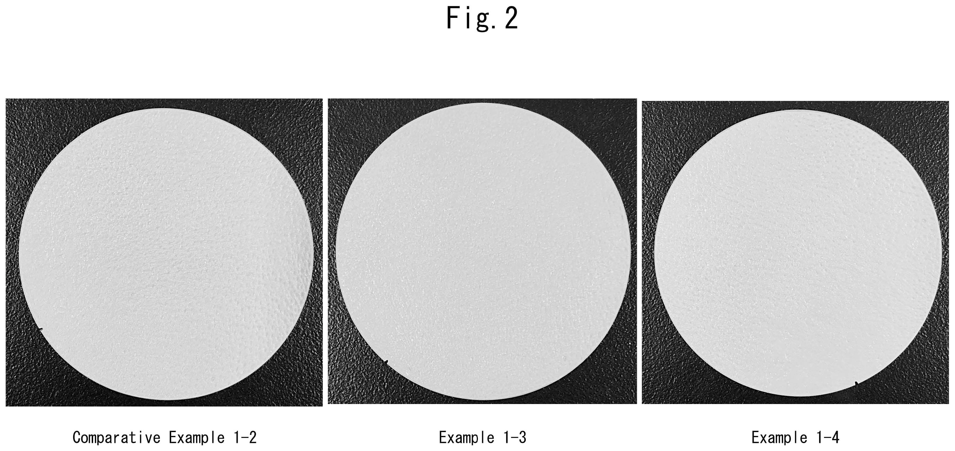

The cosmetic compositions of the above Examples 1-3 and 1-4 and Comparative Example 1-2 were each applied in an amount of 2 µL/cm², and the soft focus effect was evaluated using a Beaulax pore model. The obtained results are shown in FIG. 2.

As is apparent from the results in FIG. 2, regarding the visibility of pores, Example 1-3 exhibited the least visibility of pores, i.e., the greatest soft focus effect, followed by Example 1-4, and then Comparative Example 1-2.

### [The second invention]

Synthesis Examples 1 and 2 below were synthesized in the same manner as "Synthesis Examples 1 and 2" in the experimental section of "The first invention" described above. Various physical properties of the obtained polymer 1 and polymer 2 are as described in Table 1-1 and Table 1-2 above.

### <<Examples 2-1 and 2-2 and Comparative Example 2-1>>

In Examples 2-1 and 2-2, 5% by mass aqueous solutions of polymers 1 and 2 obtained above were prepared, respectively, and the stratum corneum softening effect was evaluated. In Comparative Example 2-1, 100% water was used.

Evaluation of the stratum corneum softening effect was carried out as follows.

### <Evaluation of stratum corneum softening effect>

The stratum corneum softening effect was evaluated based on the storage modulus (E') of the stratum corneum. A lower value of the storage modulus suggests that the stratum corneum is softer, i.e., a greater stratum corneum softening effect.

More specifically, a dynamic viscoelastic analyzer DVA-220 (manufactured by IT Keisoku Seigyo Co., Ltd.), a horizontal-drive rheometer, was used. The ends of the human stratum corneum specimen, prepared by trypsin treatment, were held horizontally by two clamps mounted in the measurement unit of the apparatus. The measurement unit had a temperature- and humidity-variable chamber structure, and was set to a skin surface temperature of 32°C and a relative humidity of 50% under dry conditions. At one of the clamps, a fixed-amplitude sinusoidal stress (strain of 0.2%, 1 Hz) was applied to one end of the stratum corneum. At the other clamp, a high-sensitivity sensor that detects weak stress transmitted via the stratum corneum was installed. Detected stress signals are converted into dynamic storage modulus (E') and dynamic loss modulus (E''), of which E' was used as an indicator of stratum corneum softness. Specifically, a smaller numerical value of E' means a softer stratum corneum. By measuring the E' of stratum corneum treated with each sample, the effect of each sample on E' was evaluated.

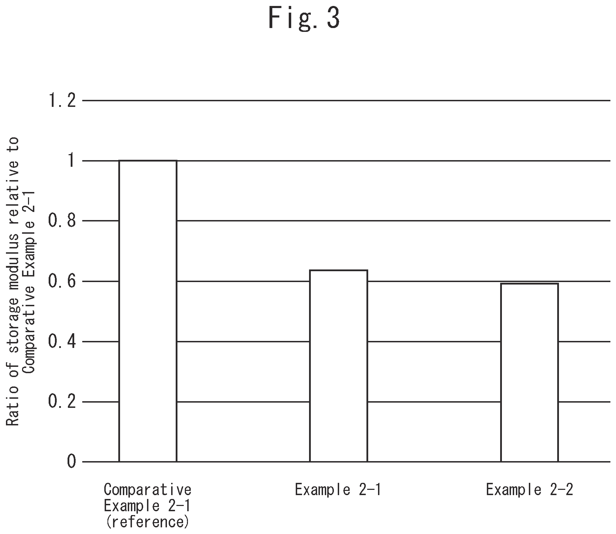

For convenience of comparison, the storage modulus value of the obtained Comparative Example 2-1 was used as a reference, and the ratio of storage modulus of each of the Examples and Comparative Example relative to Comparative Example 2-1 was determined. The obtained results are shown in FIG. 3.

As is apparent from the results in FIG. 3, it was found that Examples 2-1 and 2-2 using polymers 1 and 2, respectively, both had a notably lower storage modulus value compared to Comparative Example 2-1.

Specifically, these results suggested that polymer 1 and polymer 2 both exhibited a markedly greater softening effect on stratum corneum.

## Claims

1. A soft focus agent for skin,
wherein the soft focus agent comprises polymer (I) having a structure represented by formula (1) below as an active ingredient, or consists of the polymer (I), 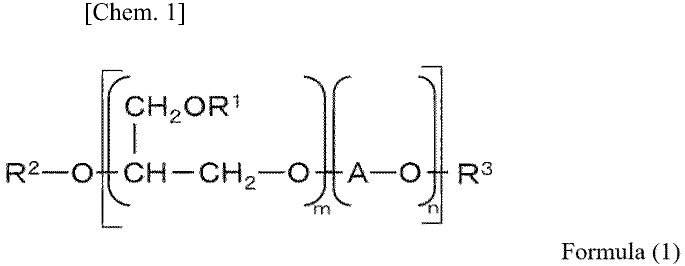
wherein, in the formula (1), R¹, R², and R³ are each independently a hydrogen atom or an alkyl group having 1 to 4 carbon atoms; A is an alkylene group having 2 to 4 carbon atoms; and m and n are each independently 1.0 to 50,
wherein number average molecular weight of the polymer (I) is 10,000 or less, and
wherein IOB value of the polymer (I) is 0.4 to 1.8.

2. The soft focus agent according to claim 1, wherein,
in the formula (1), A is represented by formula (2) below: 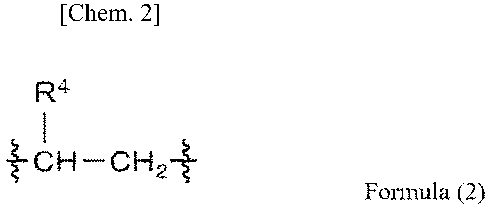
wherein, in the formula (2), R⁴ is an alkyl group having 1 or 2 carbon atoms.

3. The soft focus agent according to claim 1, wherein the polymer (I) is a random copolymer or a block copolymer.

4. The soft focus agent according to claim 1, wherein, in the formula (1), m is 2 or greater; and at least a portion of R¹ is a methyl group.

5. The soft focus agent according to claim 1, wherein, in the formula (1), at least one of R² and R³ is a methyl group.

6. A cosmetic composition, comprising the soft focus agent according to any one of claims 1 to 5.

7. A stratum corneum softening agent for skin,
wherein the stratum corneum softening agent comprises a polymer (I) having a structure represented by formula (1) below as an active ingredient, or consists of the polymer (I), 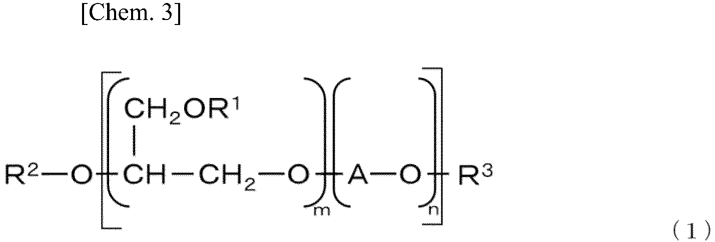
wherein, in the formula (1), R¹, R², and R³ are each independently a hydrogen atom or an alkyl group having 1 to 4 carbon atoms; A is an alkylene group having 2 to 4 carbon atoms; and m and n are each independently 1.0 to 50,
wherein number average molecular weight of the polymer (I) is 10,000 or less, and
wherein IOB value of the polymer (I) is 0.4 to 1.8.

8. The stratum corneum softening agent according to claim 7, wherein,
in the formula (1), A is represented by formula (2) below: 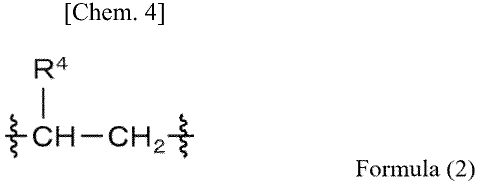
wherein, in the formula (2), R⁴ is an alkyl group having 1 or 2 carbon atoms.

9. The stratum corneum softening agent according to claim 7, wherein the polymer (I) is a random copolymer or a block copolymer.

10. The stratum corneum softening agent according to claim 7, wherein, in the formula (1), m is 2 or greater; and at least a portion of R¹ is a methyl group.

11. The stratum corneum softening agent according to claim 7, wherein, in the formula (1), at least one of R² and R³ is a methyl group.

12. A cosmetic composition, comprising the stratum corneum softening agent according to any one of claims 7 to 11.
